# EUROPEAN PATENT SPECIFICATION

(11) **EP 4 539 779 B1**
(45) Date of publication and mention of the grant of the patent: **05.08.2026**
(21) Application number: 23721170.1
(22) Date of filing: 11.04.2023
(51) Int. Cl.: A61F 2/24

(54) **FABRIC SUTURE VALVE SUSPENSION SYSTEM**
AUFHÄNGUNGSSYSTEM FÜR GEWEBENAHTVENTIL
SYSTÈME DE SUSPENSION DE VALVULE DE SUTURE EN TISSU

(30) Priority: 20.06.2022 US 202263353678 P
(43) Date of publication of application: 23.04.2025
(73) Proprietor: St. Jude Medical, Cardiology Division, Inc., St. Paul MN 55117 (US)
(72) Inventor: KING, Alec, Maple Grove, Minnesota 55369 (US)
(74) Representative: Gill Jennings & Every LLP
(86) International application number: PCT/US2023/018107
(87) International publication number: WO 2023/249682

(56) References cited:
- US-A1- 2014 277 390
- US-A1- 2018 125 649
- US-A1- 2018 206 983
- US-A1- 2018 235 756
- US-A1- 2022 175 522

## Description

### Cross-Reference to Related Applications

This application claims benefit to the filing date of U.S. Provisional Patent Application No. 63/353,678, filed June 20, 2022.

### Background of the Disclosure

The heart has four native valves, including the aortic valve, the pulmonary valve, the mitral valve (also known as the left atrioventricular valve) and the tricuspid valve (also known as the right atrioventricular valve). When these valves begin to fail, for example by not fully coapting and allowing retrograde blood flow (or regurgitation) across the valve, it may be desirable to repair or replace the valve. Prosthetic replacement heart valves may be surgically implanted via an open chest, open-heart procedure while the patient is on cardiopulmonary bypass. However, such procedures are extremely invasive, and frail patients, who may be the most likely to need a prosthetic heart valve, may not be likely to survive such a procedure. More recently, prosthetic heart valves have been trending to less invasive procedures, including collapsible and expandable heart valves that can be delivered through the vasculature in a transcatheter procedure.

The aortic valve and the pulmonary valve typically have a relatively circular shape and a relatively small diameter compared to the left and right atrioventricular valves. As a result, transcatheter prosthetic heart valves designed for the mitral and tricuspid valve may have significantly larger challenges that need to be overcome compared to transcatheter prosthetic heart valve designs for the aortic and pulmonary valves.

Another challenge in designing transcatheter prosthetic atrioventricular valves is avoiding or limiting conduction disturbances. When a transcatheter prosthetic atrioventricular valve is expanded into the native mitral or tricuspid valve, the device may press against tissue and result in disturbances of the natural conduction system of the heart. Because of this, pacemakers are frequently implanted along with prosthetic atrioventricular valves in order to help override any such conduction disturbances.

US 2014/277390 A1 relates to expandable prostheses such as replacement heart valves, such as for the mitral valve, that are configured to atraumatically grasp intralumenal tissue. US 2018/206983 relates to a prosthetic mitral valve includes an anchor assembly, a strut frame, and a plurality of replacement leaflets secured to the annular strut frame. US 2018/125649 A1 relates to a heart valve system including a radially self-expandable tubular body, a valve, and a tubular fabric. US 2018/235756 A1 relates to a replacement heart valve comprising an outer valve skirt attached to a frame by different stitches, i.e. tight stitches and loose stitches.

### Brief Summary

According to one aspect of the disclosure, a prosthetic heart valve includes a collapsible and expandable frame that, in an expanded condition, includes a central portion, an atrial portion flaring radially outwardly from the central portion, and a ventricular portion flaring radially outwardly from the central portion. A prosthetic valve is housed within the frame. An outer skirt is disposed on an outer surface of the frame, the outer skirt having a first end portion having a first attachment to the atrial portion or the ventricular portion, a second intermediate portion having a second attachment to the frame adjacent the central portion, and a third intermediate portion having a third attachment to the frame adjacent the central portion. The central portion is disposed between the second attachment and the third attachment. Upon transition of the frame from the expanded condition to the collapsed condition, the first attachment does not translate relative to the frame, while the second and third attachments do translate relative to the frame.

A center portion of the outer skirt that is positioned between the second attachment and the third attachment may be in tension when the frame is in the expanded condition and may not be in tension when the frame is in the collapsed condition. The frame may be an outer frame, and the prosthetic heart valve may include an inner frame attached to and disposed within the outer frame, the prosthetic valve being coupled to the inner frame. In another embodiment the prosthetic heart valve includes only one frame, the prosthetic valve being attached to the frame.

The first attachment may be a suture extending in a circumferential direction of the frame, the suture crossing over a strut connection where a bottom apex of a first diamond-shaped cell joins a top apex of a vertically adjacent second diamond-shaped cell. When the frame is in the collapsed condition, the suture may be configured to slide along one or more struts forming the first and second diamond-shaped cells. When the frame is in the expanded condition, the suture may be prevented from sliding along one or more struts forming the first and second diamond-shaped cells. The first attachment of the first end portion of the outer skirt may be to the atrial portion of the frame, and an opposite fourth end portion of the outer skirt may have a fourth attachment to the ventricular portion of the frame. Upon transition of the frame from the expanded condition to the collapsed condition, the fourth attachment may not translate relative to the ventricular portion of the frame. In the expanded condition of the frame, a center portion of the outer skirt positioned between the second attachment and the third attachment may include a protrusion extending around the circumference of the frame. Upon transition of the frame from the expanded condition to the collapsed condition, the protrusion may flatten as the outer skirt elongates. The first attachment of the first end portion of the outer skirt may be to the atrial portion of the frame, and an opposite fourth end portion of the outer skirt may be attached to the ventricular portion of the frame via horizontal sutures that allow translation of the fourth end portion of the outer skirt relative to the ventricular portion of the frame. The outer skirt may include at least three discrete portions, including an atrial portion disposed on the atrial portion of the frame, a ventricular portion disposed on the ventricular portion of the frame, and a center portion extending between the atrial portion of the outer skirt and the ventricular portion of the outer skirt.

Also disclosed herein is a method of implanting a prosthetic heart valve in a native heart valve annulus includes delivering the prosthetic heart valve to the native heart valve annulus while a frame of the prosthetic heart valve is in a collapsed condition within a delivery device. The prosthetic heart valve may be deployed into the native heart valve annulus so that an atrial portion of the frame is positioned on an atrial side of the native heart valve annulus, a ventricular portion of the frame is positioned on a ventricular side of the native heart valve annulus, and a suspended portion of an outer skirt of the prosthetic heart valve contacts the native heart valve annulus without the frame pressing the suspended portion of the outer skirt into the native heart valve annulus. Deploying the prosthetic heart valve may include allowing the prosthetic heart valve to transition from the collapsed condition to an expanded condition. While the prosthetic heart valve transitions from the collapsed condition to the expanded condition, the suspended portion of the outer skirt may slide relative to struts that form the frame of the prosthetic heart valve. While the prosthetic heart valve transitions from the collapsed condition to the expanded condition, the outer skirt may have a first portion rigidly coupled to frame so that the first portion does not slide relative to struts that form the frame of the prosthetic heart valve While the prosthetic heart valve transitions from the collapsed condition to the expanded condition, an outer protrusion may form in the suspended portion of the outer skirt. The suspended portion of the outer skirt may not be in tension while the prosthetic heart valve is in the collapsed condition within the delivery device. The suspended portion of the outer skirt may be in tension after the prosthetic heart valve transitions from the collapsed condition to the expanded condition.

### Brief Description of the Drawings

Fig. 1 is a schematic illustration of the right atrioventricular valve.
Fig. 2A is a side view of an assembled stent frame of a prosthetic heart valve according to an embodiment of the disclosure, the stent frame being shown in an expanded condition.
Fig. 2B is a side view of an outer frame of the stent frame of Fig. 2A.
Fig. 2C is a flattened view of the outer stent of Fig. 2B, as if cut longitudinally and laid out flat on a table in an unexpanded condition.
Fig. 2D is a side view of an inner frame of the stent frame of Fig. 2A.
Fig. 2E is a flattened view of the inner stent of Fig. 2D, as if cut longitudinally and laid out flat on a table in an unexpanded condition.
Fig. 3A is a cross-section of a prosthetic heart valve according to another aspect of the disclosure.
Fig. 3B is a plan view of a suture pattern connecting the outer skirt to the outer frame of the prosthetic heart valve of Fig. 3A.
Fig. 3C is a highly schematic cross-section of a portion of the outer frame and outer skirt of the prosthetic heart valve of Fig. 3A.
Fig. 3D is a highly schematic cross-section of a portion of the outer frame and outer skirt of the prosthetic heart valve of Fig. 3A according to an alternate embodiment.
Fig. 3E is a plan view of a suture pattern connecting the outer skirt to the outer frame of the prosthetic heart valve of Fig. 3A using an alternative pattern than is shown in Fig. 3B.

### Detailed Description

As used herein, the term "inflow end," when used in connection with a prosthetic heart valve, refers to an end of the prosthetic heart valve into which blood first flows when the prosthetic heart valve is implanted in an intended position and orientation. On the other hand, the term "outflow end," when used in connection with a prosthetic heart valve, refers to the end of the prosthetic heart valve through which blood exits when the prosthetic heart valve is implanted in an intended position and orientation. In the figures, like numbers refer to like or identical parts. As used herein, the terms "substantially," "generally," "approximately," and "about" are intended to mean that slight deviations from absolute are included within the scope of the term so modified. When ranges of values are described herein, those ranges are intended to include sub-ranges. For example, a recited range of 1 to 10 includes 2, 5, 7, and other single values, as well as all sub-ranges within the range, such as 2 to 6, 3 to 9, 4 to 5, and others.

The present disclosure is generally directed to collapsible prosthetic tricuspid valves. However, it should be understood that the features described herein may apply to other types of prosthetic heart valves, including prosthetic heart valves that are adapted for use in other heart valves, such as the mitral heart valve. Further, the features of the prosthetic heart valves described herein may, in some circumstances, be suitable for surgical (*e.g*., non-collapsible) prosthetic heart valves. However, as noted above, the disclosure is provided herein in the context of a collapsible and expandable prosthetic tricuspid valve.

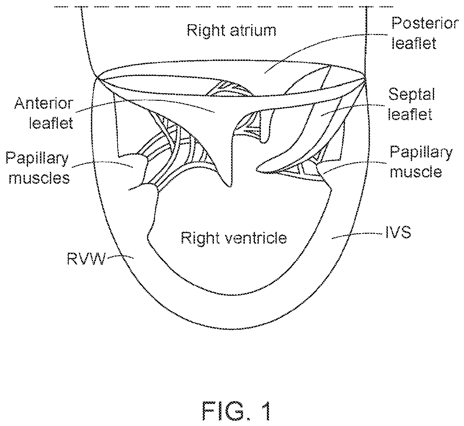

Fig. 1 is a schematic illustration of the right atrioventricular valve (commonly referred to as the tricuspid valve). The tricuspid valve separates the right atrium from the right ventricle, and typically includes three leaflets, including a posterior leaflet, an anterior leaflet, and a septal leaflet. The septal leaflet is positioned nearest the interventricular septum ("IVS"). The tricuspid valve annulus may include conduction nodes near the connection point between the annulus and the septal leaflet, including for example the atrioventricular node ("AV node"). Electrical impulses may be conducted from the AV node, via the bundle of His, to the Purkinje fibers that provide electrical conduction to the ventricles. Papillary muscles along the right ventricular wall ("RVW") may support chordae tendineae coupled to the tricuspid valve leaflets to prevent inversion of the leaflets during normal physiological operation. The left atrioventricular valve (commonly referred to as the mitral valve) may have a generally similar structure as the tricuspid valve, although many differences do exist - including for example the mitral valve typically includes two leaflets (an anterior and posterior leaflet) and has the general shape of a hyperbolic paraboloid or "saddle"-type shape. Both the mitral valve annulus and tricuspid valve annulus may be very large compared to the aortic and pulmonary valves. For example, the tricuspid valve may have a diameter of between 45-50 mm in a patient with moderate tricuspid valve disease, and a diameter of between 50-60 mm in a patient with severe tricuspid valve disease.

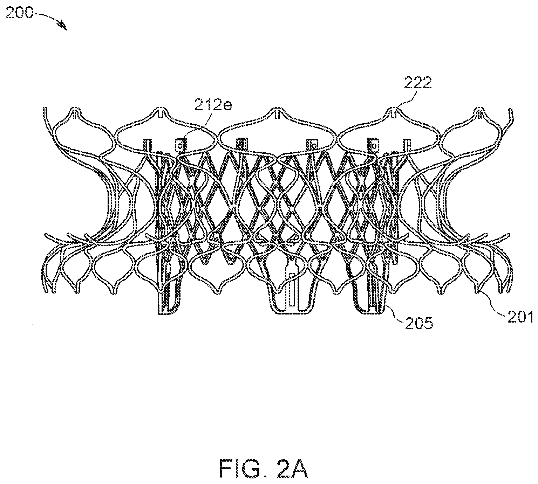

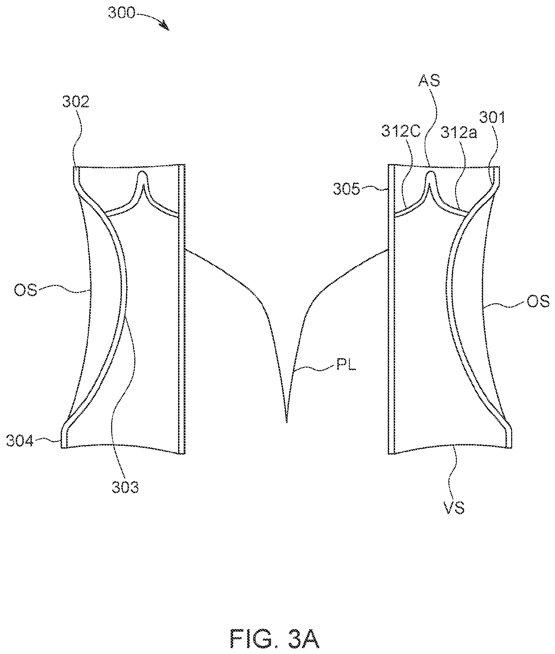

One embodiment of a prosthetic heart valve is described below in connection with Figs. 2A-E. However, it should be understood that the prosthetic heart valve described in connection with Figs. 2A-E is intended to introduce general features of a prosthetic mitral or tricuspid valve. Additional features are described in connection with Figs. 3A-C, and it should be understood that those features may be applied to the prosthetic heart valve of Figs. 2A-E, or to variations of the prosthetic heart valve of Figs. 2A-E.

Fig. 2A illustrates another embodiment of a collapsible and expandable prosthetic heart valve 200, which may be particularly suited for the replacement of a native mitral or tricuspid valve. It should be understood that the prosthetic heart valve 200 illustrated in Fig. 2A omits certain features that would typically be included, such as a valve assembly to assist in controlling blood flow through the prosthetic heart valve, and interior and/or exterior fabrics or tissue skirts to assist with providing a seal around the prosthetic heart valve and/or with enhancing tissue ingrowth to fix the prosthetic heart valve within the native heart valve over time. However, for purposes of simplicity, the prosthetic leaflet(s) and skirt(s) are omitted from the drawings for clarity of illustration. The prosthetic heart valve 200 is illustrated in Fig. 2A in an expanded configuration. The stent of the prosthetic heart valve 200 may include an outer stent or frame 201 and an inner stent or frame 205 positioned radially within the outer frame. The outer frame 201 may be primarily for anchoring the prosthetic heart valve 200 within the native heart valve annulus, while the inner frame 205 may be primarily for holding the prosthetic valve assembly in the desired position and orientation.

The outer frame 201 and/or the inner frame 205 may be formed of a superelastic and/or shape memory material such as nitinol. According to some examples, other biocompatible metals and metal alloys may be suitable. For example, superelastic and/or self-expanding metals other than nitinol may be suitable, while still other metals or metal alloys such as cobalt-chromium or stainless steel may be suitable, particularly if the stent or support structure is intended to be balloon expandable. In some examples, the outer frame 201 and/or inner frame 205 may be laser cut from one or more tubes, such as a shape memory metal tube. The shape memory metal tube may be nitinol or any other bio-compatible metal tube. For example, the outer frame 201 may be laser cut from a first tube while the inner frame 205 may be laser cut from a second tube of a smaller diameter.

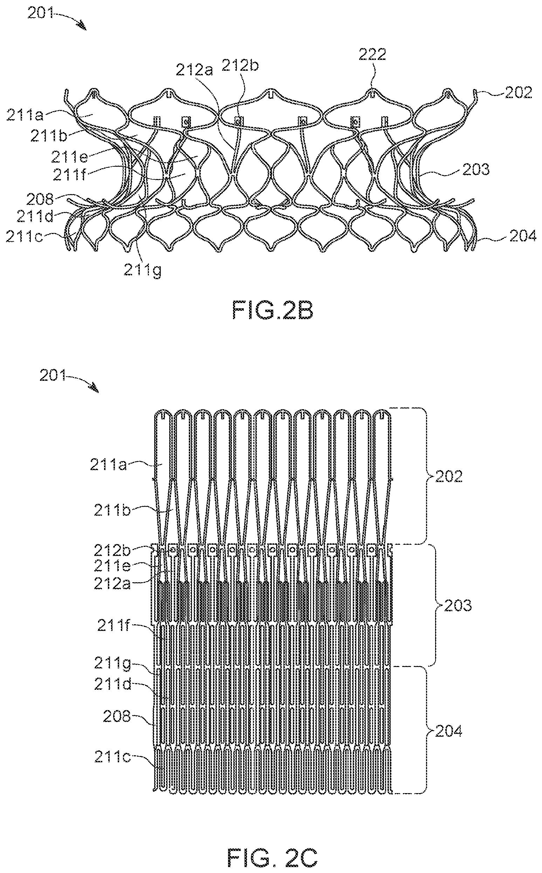

The prosthetic heart valve 200 may be adapted to expand from a collapsed or constrained configuration to an expanded configuration. According to some examples, the prosthetic heart valve 200 may be adapted to self-expand, although the prosthetic heart valve could instead be partially or fully expandable by other mechanisms, such as by balloon expansion. The prosthetic heart valve 200 may be maintained in the collapsed configuration during delivery, for example via one or more overlying sheaths that restrict the valve from expanding. The prosthetic heart valve 200 may be expanded during deployment from the delivery device once the delivery device is positioned within or adjacent the native valve annulus. In the expanded configuration, the atrial portion 202 and ventricular portion 204 may extend radially outward from a central longitudinal axis of the prosthetic heart valve 200 and/or central portion 203, and may be considered to flare outward relative to the central longitudinal axis of the replacement valve and/or central portion 203. The atrial portion 202 and ventricular portion 204 may be considered flanged relative to central portion 203. The flared configuration of atrial and ventricular portions 202, 204 relative to central portion 203 is described in the context of a side view of the outer frame 201, as can be best seen in Fig. 2B. In some embodiments, the flared configuration of the atrial and ventricular portions 202, 204 and the central portion 203 may define a general hour-glass shape in a side view of the outer frame 201. That is, the atrial and ventricular portions 202, 204 may be flared outwards relative to the central portion 203 and then curved or bent to point at least partially back in the axial direction. It should be understood, however, that an hour-glass configuration is not limited to symmetrical configuration.

Outer frame 201 is illustrated in Figs. 2B-C isolated from other components of the prosthetic heart valve 200. In Fig. 2B, the outer frame 201 is illustrated in an expanded condition. In Fig. 2C, the outer frame 201 is illustrated in an unexpanded condition, as if cut longitudinally and laid flat on a table. As used herein, the term "unexpanded" refers to the state of the stent prior to being shape-set (*e.g*., immediately after it is cut from a tube of nitinol). After being formed, the stent may be shape-set to the expanded condition and may also have a collapsed condition in which the stent is collapsed to a smaller size from its expanded condition. The shape of the stent may be similar, but not necessarily identical, in the unexpanded and collapsed conditions

Outer frame 201 may include an atrial portion or anchor 202, a ventricular portion or anchor 204, and a central portion 203 coupling the atrial portion to the ventricular portion. The atrial portion and ventricular portion may be referred to herein as atrial or ventricular disks. Atrial portion 202 may be configured and adapted to be disposed on an atrial side of a native valve annulus and may flare radially outwardly from the central portion 203. Ventricular portion 204 may be configured and adapted to be disposed on a ventricular side of the native valve annulus and may also flare radially outwardly from the central portion 203. The central portion 203 may be configured to be situated in the valve orifice, for example in contact with the native valve annulus. However, as is described in greater detail below, it may be preferable for the central portion 203 to avoid pressing against or to only minimally press against the native valve annulus after implantation. In use, the atrial portion 202 and ventricle portion 204 effectively clamp the native valve annulus on the atrial and ventricular sides thereof, respectively, holding the prosthetic heart valve 200 in place.

The atrial portion 202 may be formed as a portion of a stent or other support structure that includes or is formed by a plurality of generally diamond-shaped cells, although other suitable cell shapes, such as triangular, quadrilateral, or polygonal may be appropriate. In some examples, the atrial portion 202 may be formed as a braided mesh, as a portion of a unitary stent, or a combination thereof. According to one example, the stent that includes the atrial portion 202 may be laser cut from a tube of nitinol and heat set to the desired shape so that the stent, including atrial portion 202, is collapsible for delivery, and re-expandable to the set shape during deployment. The atrial portion 202 may be heat set into a suitable shape to conform to the native anatomy of the valve annulus to help provide a seal and/or anchoring between the atrial portion 202 and the native valve annulus. The heat-set atrial portion 202 may be partially or entirely covered by a cuff or skirt, on the luminal and/or abluminal surface of the atrial portion 202. The skirt may be formed of any suitable material, including biomaterials such as bovine pericardium, biocompatible polymers such as ultra-high molecular weight polyethylene, woven polyethylene terephthalate ("PET") or expanded polytetrafluoroethylene ("ePTFE"), or combinations thereof. The atrial portion 202 may include features for connecting the atrial portion to a delivery system. For example, the atrial portion 202 may include pins or tabs 222 around which sutures (or suture loops) of the delivery system may wrap, so that while the suture loops are wrapped around the pins or tabs 222, the outer frame 201 maintains a connection to the delivery device.

The ventricular portion 204 may also be formed as a portion of a stent or other support structure that includes or is formed of a plurality of diamond-shaped cells, although other suitable cell shapes, such as triangular, quadrilateral, or polygonal may be appropriate. In some examples, the ventricular portion 204 may be formed as a braided mesh, as a portion of a unitary stent, or a combination thereof. According to one example, the stent that includes the ventricular portion 204 may be laser cut from a tube of nitinol and heat set to the desired shape so that the ventricular portion 204 is collapsible for delivery, and re-expandable to the set shape during deployment. The ventricular portion 204 may be partially or entirely covered by a cuff or skirt, on the luminal and/or abluminal surface of the ventricular portion 204. The skirt may be formed of any suitable material described above in connection with the skirt of atrial portion 202. It should be understood that the atrial portion 202 and ventricular portion 204 may be formed as portions of a single support structure, such as a single stent or braided mesh. However, in other embodiments, the atrial portion 202 and ventricular portion 204 may be formed separately and coupled to one another.

The outer frame 201 may be configured to expand circumferentially (and radially) and foreshorten axially as the prosthetic heart valve 200 expands from the collapsed delivery configuration to the expanded deployed configuration. The outer frame 201 may define a plurality of atrial cells 211a, 211b in two circumferential rows. For example, the first row of atrial cells 211a may be generally diamond shaped and positioned on the inflow end of the outer frame 201. The second row of atrial cells 211b may be positioned at least partially between adjacent atrial cells 211a in the first row, with the atrial cells 211b in the second row being positioned farther from the inflow end than the first row of atrial cells 211a. The outer stent 201 may include twelve atrial cells 211a in the first row each having a diamond-shape, and twelve atrial cells 211b in the second row each having a skewed diamond shape. This skewed diamond shape, which is wider nearer the inflow (or top) end and narrower nearer the outflow (or bottom) end, may assist in transitioning from twelve cells per row on the atrial side of the stent to twenty-four cells per row on the ventricular side.

The outer frame 201 may include a plurality of ventricular cells 111c in a first row, and another plurality of ventricular cells 11d in a second row. The first row of ventricular cells 211c may be at the outflow end of the outer frame 201, and the second row of ventricular cells 211d may be positioned farther from the outflow end than, and adjacent to, the first row of ventricular cells 211c. In the illustrated embodiment the first and second rows of ventricular cells 211c, 211d are all generally diamond-shaped and have substantially the same, or an identical, size, with twenty-four cells in the first row of ventricular cells 211c and twenty-four cells in the second row of ventricular cells 211d.

Outer stent 201 is also illustrated as including three rows of center cells. A first row of center cells 211e is positioned adjacent the atrial end of the outer stent 201, each cell 211e being positioned between a pair of adjacent atrial cells 211b. Each center cell 211e may be substantially diamond-shaped, but it should be understood that adjacent center cells 211e do not directly touch one another. The first row of center cells 211e may include twelve center cells 211e, with the combination of atrial cells 211b and the center cells 211e helping transition from rows of twelve cells on the atrial side to rows of twenty-four cells on the ventricular side. A second row of center cells 211f may be positioned at a longitudinal center of the outer frame 201, each center cell 211f being positioned between an atrial cell 211b and center cell 211e. In the illustrated embodiment, center cells 211f in the second row may be diamond-shaped, with the second row including twenty-four center cells 211f. Finally, a third row of center cells 211g may be positioned between the second row of center cells 211f and the second row of ventricular cells 211d. The third row of center cells 211g may include twenty-four cells and they may each be substantially diamond-shaped.

All of the cells 211a-g may be configured to expand circumferentially and foreshorten axially upon expansion of the outer frame 201. A pin or tab 222 may extend from an apex of each atrial cell 211a in the first row in a direction toward the outflow end of the outer frame 201. Although one pin or tab 222 is illustrated in each atrial cell 211a in the first row, in other embodiments fewer than all of the atrial cells in the first row may include a pin or tab. These pins or tabs 222 may be configured to receive a suture or suture loop of a delivery device so that the prosthetic heart valve 200 remains coupled to the delivery system until the user decouples the suture loops from the pins or tabs 222.

Outer frame 201 may include coupling arms 212a. Each coupling arm 212a may be a strut that is coupled to a bottom or outflow apex of each atrial cell 211b in the second row, with each strut extending toward the inflow end of the outer frame 201 to a free end of the coupling arm 212a. The free end of each coupling arm 212a may include an aperture 212b for coupling to the inner frame 205, as described in greater detail below. In the collapsed condition (similar to the unexpanded condition shown in Fig. 2C), each coupling arm 212a is substantially surrounded by an atrial cell 211b in the second row. In the expanded condition, best shown in Fig. 2B, the coupling arms 212a may extend radially inwardly and have a contour so that the free end extends substantially parallel to the center longitudinal axis of the outer frame 201. In addition, outer frame 201 may include a plurality of tines or barbs 208 extending from a center portion or ventricular portion of the outer frame for piercing native tissue in the native annulus or in the native leaflets. In the illustrated embodiment, each barb 208 is connected to a ventricular cell 211d in the second row. In some embodiments, the barb 208 may be coupled to an inflow or outflow apex of each cell. In the particular illustrated embodiment, the barbs 208 are couple to ventricular cells 211d on an inflow half of the cell, on either side of the inflow apex. For example, the barb 208 in one ventricular cell 211d may be coupled to the inflow half of that cell on a right side of the apex, with the adjacent ventricular cell 211d having a barb coupled to the inflow half of that cell on a left side of the apex. With this configuration, the barbs 208 are provided in pairs with relatively little space between the barbs of a pair, but a relatively large space between adjacent pairs. However, it should be understood that the barbs 208 may in other embodiments be centered with even spacing between adjacent barbs. In the collapsed condition of the outer frame 201 (similar to the unexpanded condition shown in Fig. 2C), each barb 208 extends toward the outflow end of the outer frame, each barb being positioned within a ventricular cell 211d in the second row. In the expanded condition of the outer frame 201, as shown in Fig. 2B, the barbs 208 may hook upwardly back toward the inflow end, the barbs being configured to pierce native tissue of the valve annulus, such as the native leaflets, to help keep the prosthetic heart valve from migrating under pressure during beating of the heart. However, in some embodiments, the tines or barbs 208 may be omitted. For example, the tines or barbs 208 may be particularly helpful when used in a native mitral valve, as a prosthetic mitral valve must withstand relatively high pressures, and the tines or barbs 208 may assist with anchoring. However, the tines or barbs 208 may be omitted when the prosthetic heart valve is used as a prosthetic tricuspid valve, as pressures within the right heart are significantly lower than pressures within the left heart, and thus the tines or barbs 208 may not be needed at all for anchoring. In fact, the tines or barbs 208 may increase the likelihood of conduction disturbances, and particularly in the context of a prosthetic tricuspid valve, it may be preferable to omit the tines or barbs 208 entirely.

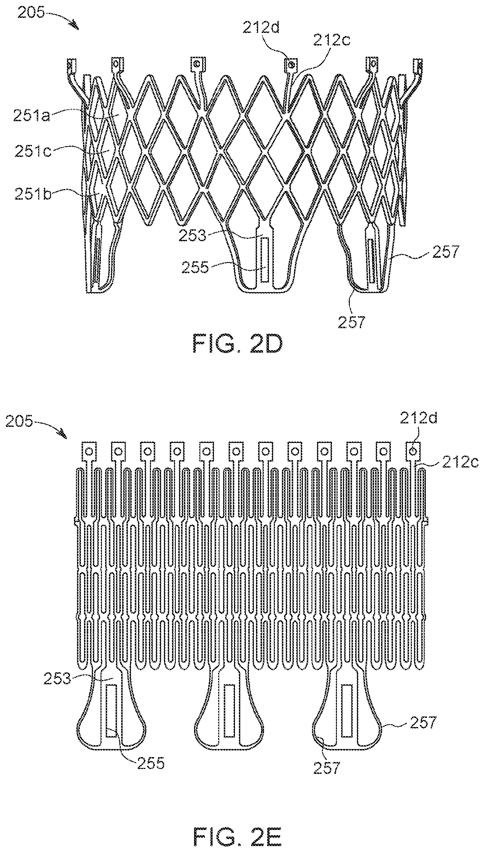

As illustrated in Fig. 2A, the inner frame 205 may be positioned radially within the outer frame 201 when the inner and outer frames are assembled together. Inner frame 205 is illustrated in Figs. 2D-E isolated from other components of the prosthetic heart valve 200. In Fig. 2D, the inner frame 205 is illustrated in an expanded condition. In Fig. 2E, the inner frame 205 is illustrated in an unexpanded condition, as if cut longitudinally and laid flat on a table. Inner frame 205 includes a plurality of rows of diamond-shaped cells so that the inner frame 205 foreshortens upon expansion. In the illustrated example, inner frame 205 includes three rows of diamond-shaped cells, including a first row of cells 251a at the inflow end of the inner frame, a second row of cells 251b at the outflow end of the inner frame, and a third row of cells 251c positioned between the first and second rows. In some embodiments, the inner frame 205 may include more or fewer rows of cells. In the expanded condition shown in Fig. 2D, the three rows of cells 251a-c may be substantially cylindrical.

One or more prosthetic leaflets may be coupled to the inner frame 205 to form a prosthetic valve assembly, the prosthetic valve assembly configured to allow unidirectional flow of blood through the prosthetic valve assembly from the atrial end toward the ventricular end of the prosthetic heart valve 200. As illustrated in Figs. 2D-E, the inner frame 205 may include a plurality of commissure windows 255 formed in axial struts 253 extending from selected cells 251b at the outflow end of the inner frame 205. For example, inner frame 205 may include three generally rectangular shaped commissure windows 255 equidistantly spaced around the circumference of the inner frame, with each commissure window adapted to provide a location for coupling two adjacent prosthetic leaflets to the axial strut 253. However, more or fewer commissure windows 255 may be provided depending on how many prosthetic leaflets will be coupled to the inner frame 205. Additional support struts 257 may connect the axial struts 253 to the cells 251b. In particular, a first support strut 257 may couple the outflow end of each axial strut 253 to the outflow apex of a first cell 251b on a first side of the axial strut, and a second support strut 257 may couple the outflow end of each axial strut 253 to the outflow apex of a second cell 251b on a second opposite side of the axial strut, with the axial strut coupled to a third cell 251b between the first and second cells. As shown in Figs. 2D-E, the support struts 257 may be contoured so as to avoid presenting any sharp tips, which may help avoid damaging the anatomy.

The inner frame 205 may also include a plurality of coupling arms 212c. Each coupling arm 212c may have a first end coupled to the inner frame 205 at an inflow end of the inner frame. In particular, the first end of each coupling arm 212c may be attached to a junction between two adjacent cells 251a in the first row at the inflow end. The coupling arms 212c may extend in a direction away from the outflow end of the inner frame 205 to a free end, with the free end including an aperture 212d therein. In the expanded condition, as shown in Fig. 2D, the coupling arms 212c may initially extend radially outwardly from the inner frame 205, with the free end being contoured so that the free end extends substantially parallel to the longitudinal axis of the inner frame 205. In the illustrated embodiment, inner frame 205 may include a total of twelve coupling arms 212c spaced equidistantly around the circumference of the inner frame. Preferably, the number of coupling arms 212c corresponds to the number of coupling arms 212a. Referring back to Fig. 2A, a coupling member, such as a suture or a rivet 212e, may pass through apertures 212b and 212d to couple the outer frame 201 to the inner frame 205.

As noted above in connection with the description of Fig. 1, the proximity of the native tricuspid valve annulus to native electrical conduction pathways of the heart creates a risk that a prosthetic tricuspid valve will cause disturbances in the electrical conduction system of the heart. Thus, it may be desirable to minimize (or more evenly distribute) the amount of radial force that a prosthetic tricuspid valve exerts on the native tricuspid annulus upon implantation, as well as to limit the amount of contact between rigid structure of the prosthetic heart valve and the tricuspid valve annulus. In the context of a prosthetic tricuspid valve, the structures that are rigid are typically the metal frames, such as the central portion 203 of the prosthetic heart valve 200. By limiting metal-on-annulus contact and limiting the amount of radial force applied to the native tricuspid valve annulus, the risk of conduction disturbances (and thus the potential need for pacemaker implantation) may be reduced. One way in which to achieve both goals simultaneously is by having a fabric or tissue skirt member be in direct contact with the native tricuspid valve annulus while limiting or eliminating any direct force applied to the native tricuspid valve annulus by the stent of the prosthetic heart valve. Fabrics (which may be formed of any of the fabrics described in connection with the skirt of atrial portion 202) and tissues (for example bovine or porcine pericardium) are soft compared to a metal stent, and these skirt members will tend to exert force against the native annulus that is more broadly and uniformly distributed than metal struts pressing against the naive annulus. In known prosthetic heart valves, tissue or fabric skirts are often provided on the luminal or outer surface of a stent, but typically the stent will press the skirt into the native valve annulus. In other words, even though there may not be direct metal-to-tissue contact in these prior art devices, the problem of rigid contact with the annulus and high forces is not alleviated. But, as described below, by providing a skirt that is suspended and able to contact the native valve annulus, without the metal stent pressing the skirt into the native valve annulus, conduction disturbances may be minimized or eliminated. One potential problem with such a design is that the skirt should not significantly limit the ability of the metal frame(s) of the prosthetic heart valve to collapse and expand. For example, a skirt may be suspended on the outer frame 201 of prosthetic heart valve 200 by simply suturing one side of the skirt to the atrial portion 202, and the other side of the skirt to the ventricular portion 204, with the center of the skirt suspended between the atrial and ventricular portion and radially outside of the central portion 203. However, this configuration might entirely limit the ability of the outer stent 201 to collapse. This is because the outer stent will elongate axially upon radial collapse and foreshorten axially upon radial expansion. If an outer skirt was suspended between the atrial and ventricular disks, the presence of the skirt (and its attachment to the atrial and ventricular disk) may limit (or eliminate) the ability of the outer stent 201 to collapse without tearing the skirt or otherwise requiring significant forces. Embodiments described below may solve some or all of these potential problems while achieving the desired results relating to anchoring and avoiding disturbing the natural conduction of the heart.

Fig. 3A is a cross-section of a prosthetic heart valve 300 according to another aspect of the disclosure. Prosthetic heart valve 300 may be a prosthetic atrioventricular valve (*e.g.,* a prosthetic tricuspid valve). Prosthetic heart valve 300 may include an outer stent or frame 301 and an inner stent or frame 305. Outer frame 301 may be coupled to inner frame 305 via coupling arms 312a, 312c. Outer frame 301 and inner frame 305 may be similar or identical to outer frame 201 and inner frame 205, respectively, and are thus not described in greater detail.

Fig. 3A also shows prosthetic leaflets PL coupled to the inner frame 305, with the prosthetic leaflets PL being in a closed or coapted condition. Prosthetic heart valve 300 may include three prosthetic leaflets PL that are formed of bioprosthetic tissue, such as bovine or porcine pericardium, which allow the unidirectional flow of blood through the prosthetic heart valve 300 from the inflow (or atrial) end to the outflow (or ventricular) end. In the particular embodiment of Fig. 3A, a ventricular skirt VS extends from the inner frame 305 to the outer frame 301 at the outflow end of the prosthetic heart valve 300 to restrict blood from flowing between the inner frame 305 and outer frame 301 from the outflow side. Similarly, an atrial skirt AS extends from the inner frame 305 to the outer frame 301 at the inflow end of the prosthetic heart valve 300 to restrict blood from flowing between the inner frame 305 and outer frame 301 from the inflow wide. The atrial skirt AS and ventricular skirt VS may be formed of any of the materials described above in connection with the skirt of the atrial portion 202 of prosthetic heart valve 200.

Similar to outer frame 201, outer frame 301 may have an hourglass shape when expanded with a narrow-waisted central portion 303 from which the atrial disk 301 and ventricular disk 304 flare radially outwardly. Unlike outer frame 201, outer frame 301 includes an outer skirt OS positioned radially outwardly of the outer frame 301, with the outer skirt OS beings suspended between the atrial portion 302 and the ventricular portion 304, such that a significant gap exists between the central portion 303 of the outer frame 301 and the outer skirt OS when the prosthetic heart valve is expanded. Although Fig. 3A is a cross-section, it should be understood that the outer skirt OS preferably extends around the entire circumference of the outer frame 301. The outer skirt OS may be formed of any of the material described above in connection with the skirt of the atrial portion 202 of prosthetic heart valve 200.

Still referring to Fig. 3A, an inflow end of the outer skirt OS (toward the top of the view of Fig. 3A) is coupled to the outer frame 301 at or near atrial portion 302, and an outflow end of the outer skirt OS (toward the bottom of the view of Fig. 3A) is coupled to the outer frame 301 at or near ventricular portion 304. When the prosthetic heart valve is in the expanded condition, as shown in Fig. 3A, the outer skirt OS is under tension. When implanted into a patient, for example in a native tricuspid heart valve, the outer skirt OS directly contacts the native valve annulus but at least some space remains between the outer skirt OS and the central portion 303 of the outer frame 301 so that the outer frame 301 does not exert significant forces on the native valve annulus and so that there is not a rigid *(e.g.,* metal) structure that is pressing against the native valve annulus. In other words, the tension on the outer skirt OS may help the outer skirt OS interface with the native anatomy instead of the valve frame, reducing or minimizing localized pressures on the native valve annulus that might otherwise cause conduction system disturbances. Preferably, the outer frame 301 does not include the tines or barbs described in connection with outer frame 201.

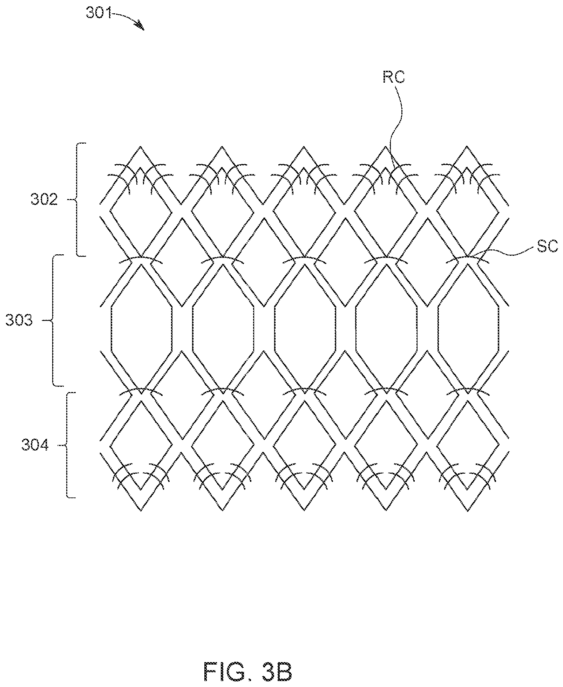

Despite the fact that the inflow and outflow ends of the outer skirt OS are coupled to the outer frame 301 to keep the outer skirt OS in tension when the prosthetic heart valve 300 is in the expanded condition, this tension is reduced or released as the prosthetic heart valve 300 collapses. As a result of this reduction or release of tension in the outer skirt OS, the prosthetic heart valve 300 is not significantly restricted from collapsing as a result of the outer skirt OS, and the forces required to load the prosthetic heart valve into a delivery device are not significantly increased by the outer skirt OS and its connection to the outer frame 301. One way to achieve this configuration in which the outer skirt OS is only under tension after deployment (or expansion) of the prosthetic heart valve 300 is shown in Figs. 3B-C.

Fig. 3B illustrates a plan view of the outer frame 301 of prosthetic heart valve 300. Generally, the outer frame 301 is formed of a plurality of diamond-shaped cells, each diamond-shaped cell being formed by four struts. As with other embodiments above, the frame may be formed via laser cutting a hollow tube of shape memory material, such as nitinol, and then shape-set into the desired hourglass shape. The outer skirt OS positioned on the outer surface of the outer frame 301 is omitted from the view of Fig. 3B, but the pattern of connections (*e.g*., sutures) that coupled to the outer skirt OS to the outer frame 301 is shown. In the illustrated embodiment, the atrial disk 302 is formed of between one and two rows of axially adjacent circumferential rows of cells, the ventricular disk 304 is formed of between one and two rows of axially adjacent circumferential rows of cells, and the central waist 303 is formed of between one and three axially adjacent circumferential rows of cells. However, it should be understood that these numbers are merely exemplary, and the features described below may work equally well with frames having collapsible cells in different shapes and numbers.

Still referring to Fig. 3B, two general types of suture connections are shown that couple the outer skirt OS (not shown in Fig. 3B) to the outer frame. A first suture connection type is a sliding connection SC. Each sliding connection SC, in this embodiment, takes the form of a suture extending horizontally (or circumferentially, which is left-to-right in the view of Fig. 3B) across a point where two vertically or axially adjacent diamond-shaped cells meet each other. In particular, sliding connections SC may be provided where the cells in the atrial portion 302 meet vertically or axially adjacent cells in the central waist portion 302. Similarly, sliding connection SC may be provided where the cells in the ventricular portion 304 meet vertically or axially adjacent cells in the central waist portion 302. With this configuration, each sliding connection SC includes a horizontal or circumferential suture that crosses over where four struts meet each other, the four struts including two struts of a first diamond-shaped cell and two struts of a vertically adjacent second diamond-shaped cell. Stated otherwise, the horizontal suture crosses where the outflow or bottom apex of one diamond-shaped cell meets the inflow or top apex of another axially or vertically adjacent diamond-shaped cell.

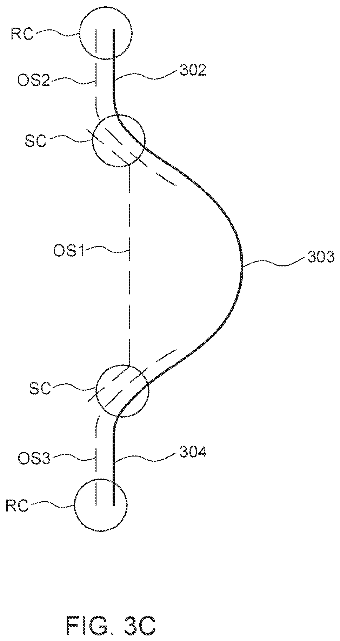

When the outer frame 301 is in an expanded condition, the cells take a diamond shape in which the horizontal or circumferential distance of each diamond is relatively large while the vertical or axial distance of each diamond is relatively small. As a result, similar to the configuration shown in Fig. 3B, the sliding connections SC are not able to slide up or down relative to the outer frame 301 because of the angle of the struts which the horizontal sutures cross over. However, as the prosthetic heart valve is collapsed (*e.g.,* for loading into a catheter for delivery to the patient), the diamond cells collapse circumferentially (in the left-right direction in the view of Fig. 3B) causing the vertical or axial apices of the cells to move apart from each other, and the horizontal or circumferential apices of the cells to draw closer to one another. As a result, the struts forming the cells tend to move closer in line with the axial or vertical direction upon collapsing of the prosthetic heart valve 300. In this condition, the horizontal sutures forming the sliding connections SC become able to slide up and down the struts that form the cells because of the steeper angle of the struts while in the collapsed condition. The ability of the sliding connections SC to slide while the prosthetic heart valve 300 is collapsed relieves the tension on the outer skirt OS. When the prosthetic heart valve 300 expands again *(e.g.,* upon deployment from the delivery catheter into the native tricuspid valve), the diamond-shaped cells expand circumferentially again such that the vertical apices of each cell draw closer to each other, while the horizontal apices of each cell move apart from each other. As the cells and the corresponding struts expand, the horizontal sutures forming the sliding connections SC are forced back to the position shown in Fig. 3B, again applying tension to the outer skirt OS. Thus, the outer skirt OS is only in tension when the prosthetic heart valve 300 is in the expanded condition. Fig. 3C is a highly schematic cross-section of one side of the outer stent 301 while in an expanded condition, showing the general locations of the sliding connections SC connecting the outer skirt OS to the outer frame 301 at the circled locations. The rigid connections RC, described below, are also generally shown in Fig. 3C.

Referring back to Fig. 3B, the second type of connections of the outer skirt OS to the outer frame 301 may be rigid connections. The rigid connections may take many forms, but generally are suture connections that do not slide (or do not significantly slide) relative to the struts of the outer frame 301, whether the outer frame 301 is collapsed or expanded. In the illustrated example, the rigid connections RC are formed as double sutures wrapping around each strut near the inflow apex of the strut (for the cells in atrial portion 302) or wrapping around each strut near the outflow apex of the strut (for the cells in the ventricular portion 304). In other words, the rigid connections RC may only be included at the terminal ends of the outer frame 301. As the outer frame 301 expands and collapses, the rigid connections RC do not move significantly relative to the struts over which they cross, and thus the outer skirt OS at these locations does not move significantly relative to the outer frame 301.

Referring again to Fig. 3C, the outer skirt OS may be formed as multiple discrete pieces. If the outer skirt OS was formed as a single integral member that was not capable of stretching or otherwise elongating, the rigid connections RC at the terminal ends of the outer skirt OS might prevent the outer frame 301 from collapsing. As shown in Fig. 3C, the outer skirt OS may include a center skirt portion OS1 that generally aligns with the central waist 303 of the outer frame 301. The outer skirt OS may also include an atrial skirt portion OS2 and a ventricular skirt portion OS3, with all three outer skirt potions being formed as discrete elements. In the illustrated embodiment, the atrial skirt portion OS2 extends along the outer frame 301 from the atrial end to a location slightly beyond the point where the atrial portion 302 begins to transition to the central waist 303. Similarly, the ventricular skirt portion OS3 extends along the outer frame 301 from the ventricular end 304 to a location slightly beyond the point where the ventricular portion 304 begins to transition to the central waist 303. The central skirt portion OS1 of the outer skirt OS has a first end that overlaps the bottom or outflow end of the atrial skirt portion OS2, and a second end that overlaps the top or inflow end of the ventricular skirt portion OS3. The extent over overlap may be smaller or greater than what is shown in Fig. 3C, and although the atrial skirt portion OS2 and ventricular skirt portion OS3 are shown as being positioned between the outer frame 301 and the central skirt portion OS1, either or both of the atrial skirt portion OS2 and ventricular skirt portion OS3 may be positioned outside of the central skirt portion OS1. With the configuration shown and described in connection with Fig. 3C, as the outer frame 301 collapses radially and elongates axially, the ends of the central skirt portion OS1 may slide relative to the outer frame 301 via the sliding connections, allowing the outer frame 301 to collapse without being significantly hindered by the connection of the central skirt portion OS1 to the outer frame 301.

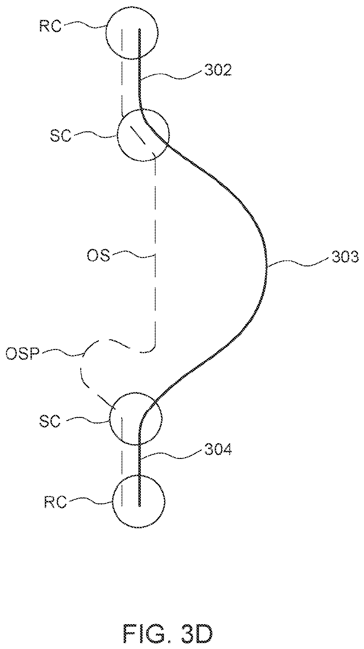

Other mechanisms may be suitable to allow for the outer skirt OS to achieve the desired functionality described above. For example, Fig. 3D is a highly schematic cross-section of an alternate embodiment of what is shown in Fig. 3C. In Fig. 3D, a single continuous outer skirt OS extends from the atrial portion 302 of the outer frame 301 to the ventricular portion 304 of the outer frame 301, with rigid connections RC at or near the terminal axial ends of the outer frame 301, and sliding connections SC in similar positions as described in connection with Fig. 3D. However, the outer skirt OS may form a bump or an outer skirt protrusion OSP that extends circumferentially around the outer frame 301. Similarly shaped skirt bumps or protrusions are described in greater detail in U.S. Patent Application No. 17/548,984, filed December 13, 2021. In some embodiments, the outer skirt protrusion OSP may be formed by heat-treating the outer skirt OS to have the desired shape, and the outer skirt protrusion OSP may generally press into the native annulus tissue to help provide improved sealing against paravalvular leak. As with the embodiment(s) above, when the outer frame 301 is radially expanded, the sliding connections SC allow for the outer skirt OS (and particularly the center portion that is radially outward of the central waist 303) to be in tension, with the outer skirt protrusion OSP tending to take the set shape. However, when the outer frame 301 radially collapses and extends elongates axially, the outer skirt protrusion OSP may tend to unfurl or straighten while the sliding connections SC slide relative to the struts of the outer frame 301.

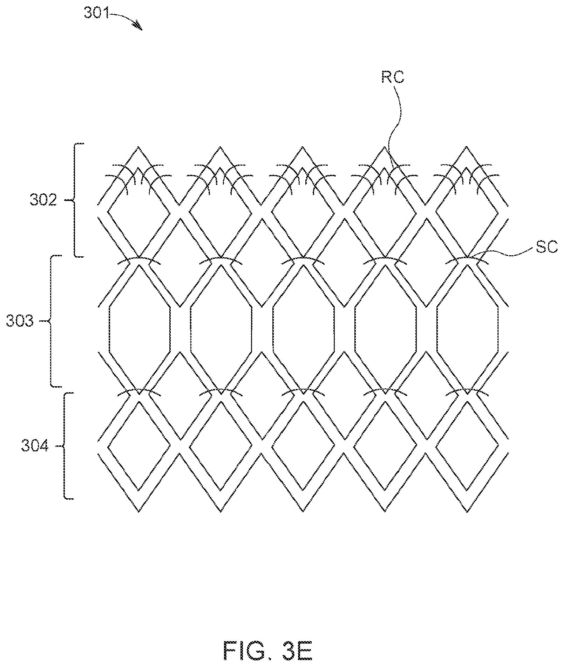

In another embodiment, shown in Fig. 3E, the suture pattern of the outer skirt OS to the outer frame 301 may be substantially identical to that shown in Fig. 3B, with one significant change. In the embodiment shown in Fig. 3E, the sliding connections SC are identical to those shown in Fig. 3B, but the rigid connections RC are only provided on one end of the outer frame. In this embodiment, the rigid connections RC are provided on the atrial portion 302 of the outer frame 301, but in other embodiments, the rigid connections may be provided on the ventricular portion 304 of the outer frame 301. In either case, the end of the outer frame 301 without the rigid connections may have no connections at all. In this embodiment, the ventricular end of the outer skirt OS would be mostly free-floating below the horizontal sutures that form the sliding connections SC. However, it may not always be desired for the ventricular end of the outer skirt OS to be free-floating. Thus, instead of omitting any connections, additional horizontal sutures similar to those shown in connection with the other sliding connections SC may be used on the ventricular end of the outer skirt OS to limit or prevent the ventricular end of the skirt from being free-floating, but while avoiding a highly rigid connection. Either way, as a result, as the outer frame 301 radially collapses and elongates, the unattached end of the outer skirt OS will not hinder the outer frame 301 from collapsing, but when the outer frame 301 expands, the sliding connections SC will maintain the outer skirt OS (and particularly the center portion of the outer skirt radially outward of the central waist 303) in tension.

The outer skirts described herein and their connection(s) to an outer frame are described above in the context of a double-stented or double-framed prosthetic heart valve with a separate outer anchoring frame and inner valve-attachment frame. However, it should be understood that the outer skirts described herein and their connection(s) to a frame may work with a single-frame prosthetic heart valve that has an hourglass-type shape generally similar to the outer frames described above.

## Claims

1. A prosthetic heart valve (300) comprising:
a collapsible and expandable frame (301) that, in an expanded condition, includes a central portion (303), an atrial portion (302) flaring radially outwardly from the central portion, and a ventricular portion (304) flaring radially outwardly from the central portion (303);
a prosthetic valve (PL) housed within the frame (301); and
an outer skirt (OS) disposed on an outer surface of the frame (301), the outer skirt (OS) having a first end portion having a first attachment (RC) to the atrial portion (302) or the ventricular portion (304),
**characterized in that** the outer skirt (OS) further comprises a second intermediate portion having a second attachment (SC) to the frame (301) adjacent the central portion (303), and a third intermediate portion having a third attachment (SC) to the frame (301) adjacent the central portion (303), the central portion (303) being disposed between the second attachment (SC) and the third attachment (SC),
wherein upon transition of the frame (301) from the expanded condition to the collapsed condition, the first attachment (RC) does not translate relative to the frame (301), while the second and third attachments (SC) do translate relative to the frame (301).

2. The prosthetic heart valve (300) of claim 1, wherein a center portion of the outer skirt (OS) positioned between the second attachment (SC) and the third attachment (SC) is in tension when the frame (301) is in the expanded condition and is not in tension when the frame (301) is in the collapsed condition.

3. The prosthetic heart valve (300) of claim 1, wherein the frame (301) is an outer frame (301), and the prosthetic heart valve includes an inner frame (305) attached to and disposed within the outer frame (301), the prosthetic valve (PL) being coupled to the inner frame (305).

4. The prosthetic heart valve (300) of claim 1, wherein the prosthetic heart valve (300) includes only one frame (301), the prosthetic valve (PL) being attached to the frame (301).

5. The prosthetic heart valve (300) of claim 1, wherein the first attachment (RC) is a suture extending in a circumferential direction of the frame (301), the suture crossing over a strut connection where a bottom apex of a first diamond-shaped cell joins a top apex of a vertically adjacent second diamond-shaped cell.

6. The prosthetic heart valve (300) of claim 5, wherein when the frame (301) is in the collapsed condition, the suture is configured to slide along one or more struts forming the first and second diamond-shaped cells.

7. The prosthetic heart valve (300) of claim 6, wherein when the frame (301) is in the expanded condition, the suture is prevented from sliding along one or more struts forming the first and second diamond-shaped cells.

8. The prosthetic heart valve (300) of claim 1, wherein the first attachment (RC) of the first end portion of the outer skirt (OS) is to the atrial portion (302) of the frame, and an opposite fourth end portion of the outer skirt (OS) has a fourth attachment (RC) to the ventricular portion of the frame (304).

9. The prosthetic heart valve (300) of claim 8, wherein upon transition of the frame (301) from the expanded condition to the collapsed condition, the fourth attachment (RC) does not translate relative to the ventricular portion (304) of the frame (301).

10. The prosthetic heart valve (300) of claim 9, wherein in the expanded condition of the frame (301), a center portion of the outer skirt (OS) positioned between the second attachment (SC) and the third attachment (SC) includes a protrusion extending around the circumference of the frame (301).

11. The prosthetic heart valve (300) of claim 10, wherein upon transition of the frame (301) from the expanded condition to the collapsed condition, the protrusion flattens as the outer skirt (OS) elongates.

12. The prosthetic heart valve (300) of claim 1, wherein the first attachment (RC) of the first end portion of the outer skirt (OS) is to the atrial portion (302) of the frame (301), and an opposite fourth end portion of the outer skirt (OS) attached to the ventricular portion (304) of the frame via horizontal sutures that allow translation of the fourth end portion of the outer skirt (OS) relative to the ventricular portion (304) of the frame (301).

13. The prosthetic heart valve (300) of claim 1, wherein the outer skirt (OS) includes at least three discrete portions (OS1, OS2, OS3), including an atrial portion (OS2) disposed on the atrial portion (302) of the frame (301), a ventricular portion (OS3) disposed on the ventricular portion (304) of the frame (301), and a center portion (OS1) extending between the atrial portion (OS2) of the outer skirt (OS) and the ventricular portion (OS3) of the outer skirt (OS).

## Patentansprüche

1. Herzklappenventil (300), umfassend:
einen einklappbaren und ausklappbaren Rahmen (301), der in einem ausgeklappten Zustand einen zentralen Abschnitt (303), einen Vorhofabschnitt (302), der sich von dem zentralen Abschnitt radial nach außen erweitert, und einen Ventrikelabschnitt (304), der sich von dem zentralen Abschnitt (303) radial nach außen erweitert, einschließt;
ein Klappenventil (PL), das in dem Rahmen (301) untergebracht ist; und
eine Außenmanschette (OS), die an einer Außenfläche des Rahmens (301) angeordnet ist, wobei die Außenmanschette (OS) einen ersten Endabschnitt mit einer ersten Befestigung (RC) an dem Vorhofabschnitt (302) oder dem Ventrikelabschnitt (304) aufweist,
**dadurch gekennzeichnet, dass** die Außenmanschette (OS) weiter einen zweiten Zwischenabschnitt mit einer zweiten Befestigung (SC) an dem Rahmen (301) benachbart zu dem zentralen Abschnitt (303) und einen dritten Zwischenabschnitt mit einer dritten Befestigung (SC) an dem Rahmen (301) benachbart zu dem zentralen Abschnitt (303) umfasst, wobei der zentrale Abschnitt (303) zwischen der zweiten Befestigung (SC) und der dritten Befestigung (SC) angeordnet ist,
wobei beim Übergang des Rahmens (301) vom ausgeklappten Zustand in den eingeklappten Zustand sich die erste Befestigung (RC) in Bezug auf den Rahmen (301) nicht verschiebt, während sich die zweite und die dritte Befestigung (SC) in Bezug auf den Rahmen (301) verschieben.

2. Herzklappenventil (300) nach Anspruch 1, wobei ein Mittelabschnitt der Außenmanschette (OS), der zwischen der zweiten Befestigung (SC) und der dritten Befestigung (SC) angeordnet ist, unter Spannung steht, wenn sich der Rahmen (301) im ausgeklappten Zustand befindet, und nicht unter Spannung steht, wenn sich der Rahmen (301) im eingeklappten Zustand befindet.

3. Herzklappenventil (300) nach Anspruch 1, wobei es sich bei dem Rahmen (301) um einen Außenrahmen (301) handelt und das Herzklappenventil einen Innenrahmen (305) einschließt, der an dem Außenrahmen (301) befestigt und darin angeordnet ist, wobei das Klappenventil (PL) mit dem Innenrahmen (305) verbunden ist.

4. Herzklappenventil (300) nach Anspruch 1, wobei das Herzklappenventil (300) nur einen Rahmen (301) einschließt, wobei das Klappenventil (PL) an dem Rahmen (301) befestigt ist.

5. Herzklappenventil (300) nach Anspruch 1, wobei die erste Befestigung (RC) eine Naht ist, die sich in einer Umfangsrichtung des Rahmens (301) erstreckt, wobei die Naht eine Strebenverbindung kreuzt, an der ein unterer Scheitelpunkt einer ersten rautenförmigen Zelle mit einem oberen Scheitelpunkt einer vertikal benachbarten zweiten rautenförmigen Zelle zusammenläuft.

6. Herzklappenventil (300) nach Anspruch 5, wobei, wenn sich der Rahmen (301) im eingeklappten Zustand befindet, die Naht dazu ausgebildet ist, entlang einer oder mehrerer Streben zu gleiten, die die erste und die zweite rautenförmige Zelle bilden.

7. Herzklappenventil (300) nach Anspruch 6, wobei, wenn sich der Rahmen (301) im ausgeklappten Zustand befindet, die Naht daran gehindert wird, entlang einer oder mehrerer Streben zu gleiten, die die erste und die zweite rautenförmige Zelle bilden.

8. Herzklappenventil (300) nach Anspruch 1, wobei die erste Befestigung (RC) des ersten Endabschnitts der Außenmanschette (OS) an dem Vorhofabschnitt (302) des Rahmens erfolgt und ein entgegengesetzter vierter Endabschnitt der Außenmanschette (OS) eine vierte Befestigung (RC) an dem Ventrikelabschnitt des Rahmens (304) aufweist.

9. Herzklappenventil (300) nach Anspruch 8, wobei beim Übergang des Rahmens (301) vom ausgeklappten Zustand in den eingeklappten Zustand sich die vierte Befestigung (RC) in Bezug auf den Ventrikelabschnitt (304) des Rahmens (301) nicht verschiebt.

10. Herzklappenventil (300) nach Anspruch 9, wobei im ausgeklappten Zustand des Rahmens (301) ein Mittelabschnitt der Außenmanschette (OS), der zwischen der zweiten Befestigung (SC) und der dritten Befestigung (SC) positioniert ist, einen Vorsprung einschließt, der sich um den Umfang des Rahmens (301) herum erstreckt.

11. Herzklappenventil (300) nach Anspruch 10, wobei beim Übergang des Rahmens (301) vom ausgeklappten Zustand in den eingeklappten Zustand sich der Vorsprung abflacht, während sich die Außenmanschette (OS) verlängert.

12. Herzklappenventil (300) nach Anspruch 1, wobei die erste Befestigung (RC) des ersten Endabschnitts der Außenmanschette (OS) an dem Vorhofabschnitt (302) des Rahmens (301) erfolgt, und ein entgegengesetzter vierter Endabschnitt der Außenmanschette (OS) an dem Ventrikelabschnitt (304) des Rahmens über horizontale Nähte befestigt ist, die eine Verschiebung des vierten Endabschnitts der Außenmanschette (OS) in Bezug auf den Ventrikelabschnitt (304) des Rahmens (301) gestatten.

13. Herzklappenventil (300) nach Anspruch 1, wobei die Außenmanschette (OS) mindestens drei separate Abschnitte (OS1, OS2, OS3) einschließt, einschließlich eines Vorhofabschnitts (OS2), der an dem Vorhofabschnitt (302) des Rahmens (301) angeordnet ist, eines Ventrikelabschnitts (OS3), der an dem Ventrikelabschnitt (304) des Rahmens (301) angeordnet ist, und eines Mittelabschnitts (OS1), der sich zwischen dem Vorhofabschnitt (OS2) der Außenmanschette (OS) und dem Ventrikelabschnitt (OS3) der Außenmanschette (OS) erstreckt.

## Revendications

1. Prothèse (300) de valvule cardiaque comprenant :
un cadre (301) repliable et déployable qui, dans un état déployé, inclut une partie (303) centrale, une partie (302) auriculaire s'évasant radialement vers l'extérieur à partir de la partie centrale, et une partie (304) ventriculaire s'évasant radialement vers l'extérieur à partir de la partie (303) centrale ;
une prothèse de valvule (PL) logée à l'intérieur du cadre (301) ; et
une jupe extérieure (OS) disposée sur une surface extérieure du cadre (301), la jupe extérieure (OS) présentant une première partie d'extrémité présentant une première fixation (RC) à la partie (302) auriculaire ou à la partie (304) ventriculaire,
**caractérisée en ce que** la jupe extérieure (OS) comprend en outre une deuxième partie intermédiaire présentant une deuxième fixation (SC) au cadre (301) adjacente à la partie (303) centrale, et une troisième partie intermédiaire présentant une troisième fixation (SC) au cadre (301) adjacente à la partie (303) centrale, la partie (303) centrale étant disposée entre la deuxième fixation (SC) et la troisième fixation (SC),
dans laquelle, lors d'un passage du cadre (301) de l'état déployé à l'état replié, la première fixation (RC) ne se déplace pas par translation par rapport au cadre (301), tandis que les deuxième et troisième fixations (SC) se déplacent bien par translation par rapport au cadre (301).

2. Prothèse (300) de valvule cardiaque selon la revendication 1, dans laquelle une partie centrale de la jupe extérieure (OS) positionnée entre la deuxième fixation (SC) et la troisième fixation (SC) est sous tension lorsque le cadre (301) se trouve dans l'état déployé et n'est pas sous tension lorsque le cadre (301) se trouve dans l'état replié.

3. Prothèse (300) de valvule cardiaque selon la revendication 1, dans laquelle le cadre (301) est un cadre (301) extérieur, et la prothèse de valvule cardiaque inclut un cadre (305) intérieur fixé à et disposé à l'intérieur du cadre (301) extérieur, la prothèse de valvule (PL) étant couplée au cadre (305) intérieur.

4. Prothèse (300) de valvule cardiaque selon la revendication 1, dans laquelle la prothèse (300) de valvule cardiaque inclut seulement un cadre (301), la prothèse de valvule (PL) étant fixée au cadre (301).

5. Prothèse (300) de valvule cardiaque selon la revendication 1, dans laquelle la première fixation (RC) est une suture s'étendant dans une direction circonférentielle du cadre (301), la suture passant par-dessus une liaison d'entretoises où un sommet inférieur d'une première cellule en forme de diamant rejoint un sommet supérieur d'une deuxième cellule en forme de diamant verticalement adjacente.

6. Prothèse (300) de valvule cardiaque selon la revendication 5, dans laquelle, lorsque le cadre (301) se trouve dans l'état replié, la suture est configurée pour coulisser le long d'une ou de plusieurs entretoises formant les première et deuxième cellules en forme de diamant.

7. Prothèse (300) de valvule cardiaque selon la revendication 6, dans laquelle, lorsque le cadre (301) se trouve dans l'état déployé, la suture est empêchée de coulisser le long d'une ou de plusieurs entretoises formant les première et deuxième cellules en forme de diamant.

8. Prothèse (300) de valvule cardiaque selon la revendication 1, dans laquelle la première fixation (RC) de la première partie d'extrémité de la jupe extérieure (OS) est à la partie (302) auriculaire du cadre, et une quatrième partie d'extrémité opposée de la jupe extérieure (OS) présente une quatrième fixation (RC) à la partie (304) ventriculaire du cadre.

9. Prothèse (300) de valvule cardiaque selon la revendication 8, dans laquelle, lors d'un passage du cadre (301) de l'état déployé à l'état replié, la quatrième fixation (RC) ne se déplace pas par translation par rapport à la partie (304) ventriculaire du cadre (301).

10. Prothèse (300) de valvule cardiaque selon la revendication 9, dans laquelle, dans l'état déployé du cadre (301), une partie centrale de la jupe extérieure (OS) positionnée entre la deuxième fixation (SC) et la troisième fixation (SC) inclut une saillie s'étendant autour de la circonférence du cadre (301).

11. Prothèse (300) de valvule cardiaque selon la revendication 10, dans laquelle, lors d'un passage du cadre (301) de l'état déployé à l'état replié, la saillie s'aplatit à mesure que la jupe extérieure (OS) s'allonge.

12. Prothèse (300) de valvule cardiaque selon la revendication 1, dans laquelle la première fixation (RC) de la première partie d'extrémité de la jupe extérieure (OS) est à la partie (302) auriculaire du cadre (301), et une quatrième partie d'extrémité opposée de la jupe extérieure (OS) fixée à la partie (304) ventriculaire du cadre par l'intermédiaire de sutures horizontales qui permettent une translation de la quatrième partie d'extrémité de la jupe extérieure (OS) par rapport à la partie (304) ventriculaire du cadre (301).

13. Prothèse (300) de valvule cardiaque selon la revendication 1, dans laquelle la jupe extérieure (OS) inclut au moins trois parties (OS1, OS2, OS3) discrètes, incluant une partie (OS2) auriculaire disposée sur la partie (302) auriculaire du cadre (301), une partie (OS3) ventriculaire disposée sur la partie (304) ventriculaire du cadre (301), et une partie (OS1) centrale s'étendant entre la partie (OS2) auriculaire de la jupe extérieure (OS) et la partie (OS3) ventriculaire de la jupe extérieure (OS).
